# EUROPEAN PATENT APPLICATION

(11) **EP 4 803 096 A1**
(43) Date of publication of application: **09.09.2026**
(21) Application number: 24886345.8
(22) Date of filing: 01.11.2024
(51) Int. Cl.: A61K 38/33, A61P 35/00

(54) **COMPOSITION FOR PREVENTION OR TREATMENT OF BLADDER CANCER**

(30) Priority: 02.11.2023 KR 20230149895
(71) Applicant: Genomictree, Inc., Daejeon 34027 (KR)
(72) Inventor: AN, Sungwhan, Daejeon 34125 (KR); OH, Taejeong, Daejeon 34127 (KR)
(74) Representative: Mewburn Ellis LLP
(86) International application number: PCT/KR2024/017038
(87) International publication number: WO 2025/095674

(57) **Abstract**

The present invention relates to a composition for the prevention or treatment of bladder cancer, and particularly, to a composition for the prevention or treatment of cancer, comprising a nucleic acid that encodes proenkephalin (PENK) protein, which is a cancer inhibitory protein inhibited by methylation in cancer cells, in particular, PENK protein inhibited by methylation in bladder cancer cells. Thus, the composition inhibits the proliferation of bladder cancer cells.

## Description

### [Technical Field]

The present invention relates to a composition for preventing or treating bladder cancer, and more particularly, to a composition for preventing or treating bladder cancer that contains a nucleic acid encoding a tumor suppressor protein, PENK (proenkephalin), which is suppressed by methylation in cancer cells, particularly a PENK protein that is suppressed by methylation in bladder cancer cells, and thus inhibits the proliferation of bladder cancer cells.

### [Background Art]

Gene therapy has been developed as therapeutic agents for treating or preventing diseases by adding new information to existing genes, modifying the genes with the information or deleting the genes, and delivering the resulting genes to the cells of patients in the form of plasmid DNA (pDNA) or mRNA (messenger RNA) (Dunbar, C. E et al., Science 2018 1:12).

The gene therapy aims to cure diseases by correcting genetic defects in patients who suffer from genetic diseases or rare diseases. Recent technological innovations and investments in research and development have brought about growth of the global gene therapy market at an average annual rate of 10%, and gene therapy is emerging as a new blue chip in the pharmaceutical market. Thereamong, mRNA is emerging as a promising therapeutic means in the field of vaccine development and protein replacement therapy. Gene therapy is based on the principle in which genes are introduced into cells in the form of pDNA or mRNA using viruses, liposomes, anti-sense technology, and the like to allow the genes to act as therapeutics (Dunbar, C. E et al., Science 2018 1:12).

mRNA therapeutics are advantageously superior to DNA or virus therapeutics in terms of stability, efficiency, and productivity. First, mRNA occurs in the cytoplasm and thus has a low probability of mutation due to infection or genomic DNA insertion. Second, mRNA may be modified in various forms within the cytoplasm and thus can improve intracellular stability by controlling the half-life or increasing the amount of translated protein. Third, mRNA is suitable for *in vitro* experiments and thus causes rapid development and GMP production and is advantageously suitable for mass-production (Wang, Y., Su et al. Molecular therapy 2013 358-367).

mRNA refers to RNA that transmits the genetic information of DNA to ribosomes and causes protein expression through translation by mRNA. Recombinant mRNA for developing therapeutic agents or vaccines is produced from linear DNA using a promoter such as T7 or SP6 (but is not limited thereto) and an RNA polymerase, and is then 5' capped and poly A adenylated (poly A tailed) by *in vitro* transcription. The mRNA thus produced has a structure similar to the mature mRNA in the cytoplasm and consists of 5' capping, 5' UTR (untranslated region), 3' UTR, poly A, and a target gene to be expressed. Thereamong, the untranslated region (5' or 3' untranslated region) affects the stability and translational activation of mRNA depending on the sequence, thus increasing the half-life and expression level. In addition, Poly A prevents the degradation of mRNA in the cytoplasm, increasing stability and expression level. Therefore, in order for mRNA therapeutics to efficiently and stably express proteins in the body, it is important to find the optimal untranslated region sequence and poly A length (Pardi, N et al,. Nature reviews 2018 261-279).

Meanwhile, extensive research has been conducted for the treatment of cancer, which accounts for the highest proportion of causes of human mortality. Conventionally known cancer treatment methods include surgery, radiotherapy, chemotherapy, immunotherapy, and gene therapy. Among these, chemotherapeutic anticancer agents have severe side effects, while radiotherapy involves excessive costs and time consumption and exposes patients to risks associated with radioactive isotopes. In addition, immunotherapy has the disadvantage of relatively low therapeutic efficiency. Accordingly, in the course of anticancer therapy research, gene therapy, a new treatment method that selectively introduces and expresses genes or proteins for cancer treatment into cancer cells and cancer tissue cells, is emerging as a promising alternative treatment modality. The principle of gene therapy involves delivering genes in the form of plasmid DNA (pDNA), PCR products, or mRNA into cells using vectors such as viruses, liposomes, or antisense technologies, thereby allowing the genes to function as therapeutic agents (Dunbar, C. E. et al., Science, 2018, 1:12). Among these, mRNA therapeutics offer advantages over DNA-based or viral therapeutics in terms of stability, efficiency, and productivity.

Epigenetics refers to phenomena in which functional changes, such as alterations in gene expression, occur without changes in the DNA base sequence, through mechanisms including DNA methylation, covalent modifications of histone proteins, and ATP-dependent chromatin remodeling. A representative example is DNA methylation, which involves the attachment of a methyl group to the cytosine residue of a CpG dinucleotide, thereby affecting the expression of various genes. Increases or decreases in DNA methylation are predominantly observed in cancer cells or tumor tissues, and extensive research is being conducted on DNA methylation of specific genes in particular cancers. Accordingly, elucidation of mechanisms regulating gene expression through DNA methylation, including the regulation of tumor suppressor genes and oncogenes, is expected to be useful not only for early cancer diagnosis but also for prognosis and personalized treatment strategies (Kiselev IS et al., Acta Naturae, 2021).

Under this technical background, the present inventors have discovered that the 5' regulatory region of the PENK gene is specifically methylated in bladder cancer cells, thereby suppressing expression of the gene. In addition, the present inventors have found that when a nucleic acid encoding the PENK protein is introduced into a cell line to induce overexpression, proliferation of the bladder cancer cell line is inhibited. Based on these findings, the present invention has been completed.

### [Disclosure]

It is an object of the present invention to provide a composition, method, or use for preventing or treating bladder cancer.

In accordance with one aspect of the present invention, the above and other objects can be accomplished by the provision of a composition for preventing or treating bladder cancer containing a nucleic acid encoding a PENK (proenkephalin) protein.

In accordance with another aspect, provided is a method of preventing or treating bladder cancer including administering to a subject a nucleic acid encoding a PENK (proenkephalin) protein.

In accordance with another aspect, provided is a use of a nucleic acid encoding a PENK (proenkephalin) protein for the preparation of a composition for preventing or treating bladder cancer.

In accordance with another aspect, provided is a composition for preventing or treating bladder cancer including a nucleic acid encoding a PENK (proenkephalin) protein and a carrier.

In accordance with another aspect, provided is a method of preventing or treating bladder cancer including administering to a subject a nucleic acid encoding a PENK (proenkephalin) protein and a carrier.

In accordance with another aspect, provided is a use of a nucleic acid encoding a PENK (proenkephalin) protein and a carrier for the preparation of a composition for preventing or treating bladder cancer.

### [Description of Drawings]

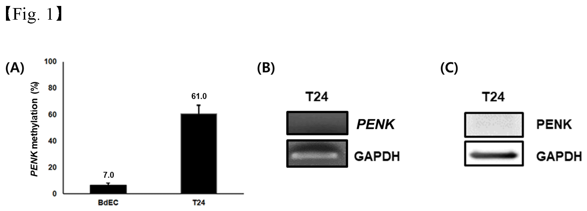

FIG. 1 shows methylation status of the promoter region of a gene encoding proenkephalin (PENK), and expression of PENK mRNA and protein ((A): measurement of methylation status of the PENK gene promoter region in normal cell lines and bladder cancer cell lines using a pyrosequencing method; (B) analysis of PENK mRNA expression in a bladder cancer cell line (T24); and (C) analysis of PENK protein expression in the bladder cancer cell line (T24).

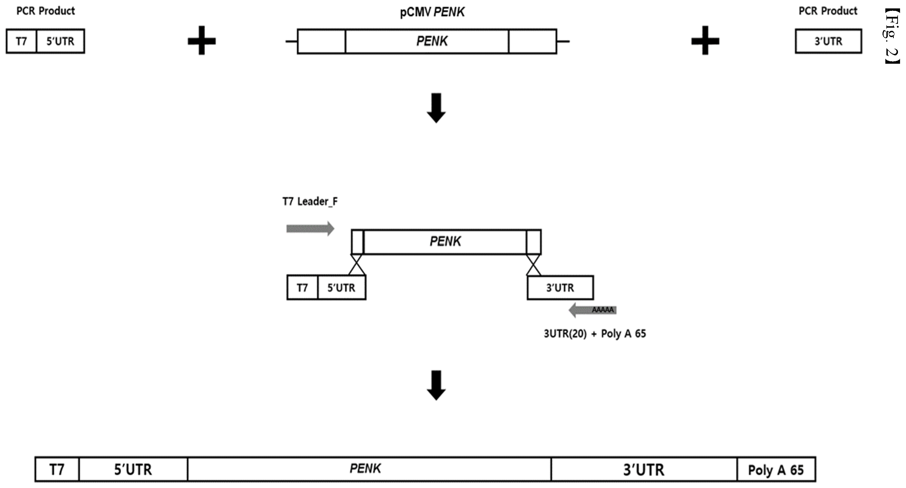

FIG. 2 illustrates a process of producing template DNA for expression of PENK gene mRNA using a PCR method.

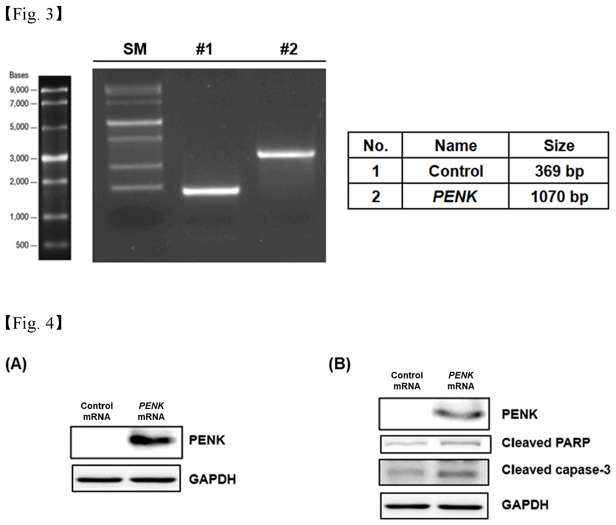

FIG. 3 shows expression of mRNA of a control group and the PENK gene confirmed by an *in vitro* transcription (IVT) method.
FIG. 4 shows expression of PENK protein and apoptosis-related factors confirmed by Western blot analysis after transfection of a bladder cancer cell line (T24) with the PENK gene mRNA produced in FIG. 3 ((A) analysis of PENK protein expression; and (B) analysis of apoptosis-inducing factors in bladder cancer cells).

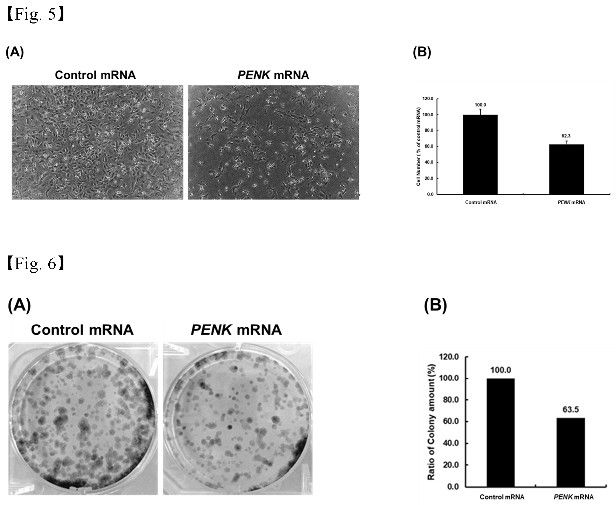

FIG. 5 shows effects of transfection of a bladder cancer cell line (T24) with the PENK gene mRNA produced in FIG. 3 on proliferation of bladder cancer cells ((A) observation of inhibition of cell proliferation by PENK mRNA using microscopy; and (B) quantitative analysis of the images presented as a graph.
FIG. 6 shows effects of transfection of a bladder cancer cell line (T24) with the PENK gene mRNA produced in FIG. 3 on colony formation of bladder cancer cells ((A) observation of inhibition of colony formation by PENK mRNA using photographic images; and (B) quantitative analysis of the images presented as a graph).

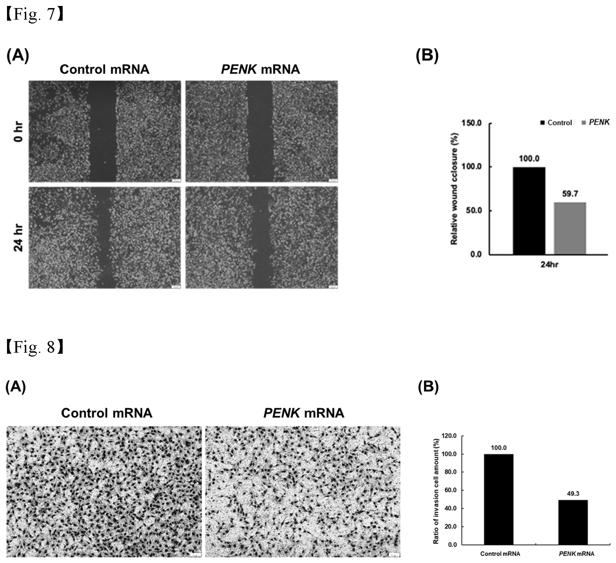

FIG. 7 shows effects of transfection of bladder cancer cell line (T24) with the PENK gene mRNA produced in FIG. 3 on wound healing ability of bladder cancer cells ((A) observation of inhibition of wound healing by PENK mRNA using microscopy; and (B) quantitative analysis of the images presented as a graph.
FIG. 8 shows effects of transfection of a bladder cancer cell line (T24) with the PENK gene mRNA produced in FIG. 3 on invasion ability of bladder cancer cells ((A) observation of inhibition of cell invasion by PENK mRNA using microscopy; and (B) quantitative analysis of the images presented as a graph.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

The present inventors have previously reported that the *PENK* gene is methylated in bladder cancer in prior research (Oh et al., BMC Cancer, 2022; Oh et al., Journal of Molecular Diagnostics, 2023). In addition, the present inventors demonstrated that *PENK* methylation acts as a biomarker for early diagnosis of bladder cancer using urine samples, and further proved that *PENK* methylation enables diagnosis of bladder cancer at an early stage even in patients with hematuria suspected of having bladder cancer.

In a specific embodiment according to the present invention, the present inventors discovered that the 5' regulatory region of the *PENK* gene is specifically methylated in bladder cancer cells, thereby suppressing expression of the gene. Furthermore, the present inventors confirmed that when a PENK mRNA-based complex is administered to a bladder cancer cell line to induce overexpression, proliferation of the bladder cancer cell line is inhibited.

Based on these findings, in one aspect, the present invention is directed to a composition for preventing or treating bladder cancer containing a nucleic acid encoding a PENK (proenkephalin) protein. In another aspect, the present invention is directed to a method of preventing or treating bladder cancer including administering to a subject a nucleic acid encoding a PENK (proenkephalin) protein. In another aspect, the present invention is directed to a use of a nucleic acid encoding a PENK (proenkephalin) protein for the preparation of a composition for preventing or treating bladder cancer.

Any nucleic acid may be used without limitation as long as it contains nucleotides as basic structural units, and the nucleic acid may include, for example, RNA or DNA.

RNA is synthesized from ribonucleotides, but in a sample containing RNA in which some of the nucleosides introduced during the synthesis are modified, RNA substantially free of errors or damage may be a gene that exhibits a desired function in an organism.

The term "DNA" refers to a polynucleotide containing a deoxyribose sugar and composed of purine or pyrimidine bases of adenine, thymine, cytosine, or guanine.

The term "RNA" refers to a polynucleotide containing a ribose sugar and typically uracil rather than thymine as one of the pyrimidine bases. RNA may contain a single-stranded molecule transcribed from DNA and have a linear sequence of nucleotide bases complementary to the transcribed DNA strand.

As used herein, the term "nucleotide" refers to a glycoside containing a sugar moiety, a base moiety, and a covalently linked internucleotide linkage, such as a phosphate or phosphorothioate internucleotide linkage. The term "nucleotide" includes both naturally occurring nucleotides, such as DNA or RNA, and non-naturally occurring nucleotides, which may optionally contain modified sugar and/or base moieties.

The term "ribonucleotide" or "deoxyribonucleotide" includes all naturally occurring and synthetic, unmodified and modified ribonucleotides or deoxyribonucleotides. Such modifications may include alterations to the sugar moiety, the base moiety, and/or the linkage between ribonucleotides or deoxyribonucleotides within an oligonucleotide.

The terms related to the nucleic acid, "polynucleotide", "nucleotide", "nucleotide sequence", and "oligonucleotide" are used interchangeably. These terms may refer to a polymeric form of nucleotides of any length, including deoxyribonucleotides or ribonucleotides, or analogs thereof. A polynucleotide may have any three-dimensional structure and may perform any known or unknown function. A polynucleotide may include one or more modified nucleotides, such as methylated nucleotides and nucleotide analogs. Modifications to the nucleotide structure may occur before or after assembly of the polymer.

As used herein, the term "polynucleotide" refers to a polymeric form of nucleotides including nucleotides of any length, including deoxyribonucleotides and/or ribonucleotides, or analogs thereof. A polynucleotide may have any three-dimensional structure and may perform any known or unknown function. The structure of a polynucleotide may be considered with reference to a 5' or 3' terminus, which indicates the directionality of the polynucleotide. In a single-stranded polynucleotide, adjacent nucleotides are generally linked by phosphodiester bonds between the 3' and 5' carbons. However, other internucleotide linkages may also be used, such as linkages including methylene or phosphoramidate bonds. Each of the 5' and 3' carbons may be exposed at opposite ends of the polynucleotide, which may be referred to as the 5' and 3' termini or simply termini. The 5' and 3' termini may also be referred to as a phosphoryl (PO₄) terminus and a hydroxyl (OH) terminus, respectively, due to the chemical groups attached to the respective termini.

The term "polynucleotide" may be used interchangeably with the term "nucleic acid" and may include both double-stranded and single-stranded molecules. Examples of the polynucleotide include, but are not limited to, genes or gene fragments (e.g., probes, primers, expressed sequence tags (ESTs), or serial analysis of gene expression (SAGE) tags), genomic DNA, fragments of genomic DNA, exons, introns, messenger RNA (mRNA), transfer RNA, ribosomal RNA, ribozymes, complementary DNA (cDNA), recombinant polynucleotides, branched polynucleotides, plasmids, vectors, isolated DNA of any sequence, isolated RNA of any sequence, nucleic acid probes, primers, or amplified copies of any of the foregoing. A polynucleotide may include modified nucleotides, such as methylated nucleotides and nucleotide analogs, including nucleotides having non-natural bases, or nucleotides having modified natural bases, such as aza- or deaza-purines.

The polynucleotide may be composed of a specific sequence of the four nucleotide bases, namely, adenine (A), cytosine (C), guanine (G), and thymine (T). Uracil (U) may be present as a natural substitute for thymine, for example when the polynucleotide is RNA. Uracil may also be used in DNA. Accordingly, the term "sequence" refers to an alphabetical representation of a polynucleotide or any nucleic acid molecule containing natural bases and/or non-natural bases.

The nucleic acid may include a sequence represented by SEQ ID NO: 1 or a sequence having at least 90% homology thereto.

[SEQ ID NO: 1]

ATGGCGCGGTTCCTGACACTTTGCACTTGGCTGCTGTTGCTCGGCCC
CGGGCTCCTGGCGACCGTGCGGGCCGAATGCAGCCAGGATTGCGCGACGTG
CAGCTACCGCCTAGTGCGCCCGGCCGACATCAACTTCCTGGCTTGCGTAATG
GAATGTGAAGGTAAACTGCCTTCTCTGAAAATTTGGGAAACCTGCAAGGAGC
TCCTGCAGCTGTCCAAACCAGAGCTTCCTCAAGATGGCACCAGCACCCTCAG
AGAAAATAGCAAACCGGAAGAAAGCCATTTGCTAGCCAAAAGGTATGGGGG
CTTCATGAAAAGGTATGGAGGCTTCATGAAGAAAATGGATGAGCTTTATCCC
ATGGAGCCAGAAGAAGAGGCCAATGGAAGTGAGATCCTCGCCAAGCGGTAT
GGGGGCTTCATGAAGAAGGATGCAGAGGAGGACGACTCGCTGGCCAATTCC
TCAGACCTGCTAAAAGAGCTTCTGGAAACAGGGGACAACCGAGAGCGTAGC
CACCACCAGGATGGCAGTGATAATGAGGAAGAAGTGAGCAAGAGATATGGG
GGCTTCATGAGAGGCTTAAAGAGAAGCCCCCAACTGGAAGATGAAGCCAAA
GAGCTGCAGAAGCGATATGGGGGCTTCATGAGAAGAGTAGGTCGCCCAGAG
TGGTGGATGGACTACCAGAAACGGTATGGAGGTTTCCTGAAGCGCTTTGCCG
AGGCTCTGCCCTCCGACGAAGAAGGCGAAAGTTACTCCAAAGAAGTTCCTG
AAATGGAAAAAAGATACGGAGGATTTATGAGATTTTAA

As used herein, the term "homology" refers to a sequence that exhibits at least 90% homology, more preferably at least 95% homology, 96% or more, 97% or more, 98% or more, or 99% or more homology, as determined by optimally aligning the sequence of the present invention with any other sequence and analyzing the aligned sequences using an algorithm commonly employed in the art. Alignment methods for sequence comparison are well-known in the art. The NCBI Basic Local Alignment Search Tool (BLAST) is accessible through NCBI or the like, and may be used in conjunction with sequence analysis programs such as BLASTP, BLASTN, BLASTX, TBLASTN and TBLASTX over the Internet. BLAST is available at www.ncbi.nlm.nih.gov/BLAST/. A method of comparing sequence homology using this program can be found at www.ncbi.nlm.nih.gov/BLAST/blast_help.html.

Based on this, the nucleic acid sequence according to the present invention may have homology of 90%, 91%, 92%, 93%, 94%, 95%, 96%, 97%, 98%, 99%, or more with the sequence disclosed herein or the entirety thereof.

The nucleic acid of the present invention may be mRNA. The mRNA according to the present invention may include the sequence of SEQ ID NO: 13.

[SEQ ID NO: 13] PENK mRNA sequence
GGGACGGCGAGGCAGGCGCUCAGAGCCCCGCAGCCUGGCCCGUGA
CCCCGCAGAGACGCUGAGGACCGCGACGAGUCGUGUCUGAACCCGGCUUU
UCCAAUUGGCCUGCUCCAUCCGAACAGCGUCAACUCCAUGGCGCGGUUC
CUGACACUUUGCACUUGGCUGCUGUUGCUCGGCCCCGGGCUCCUGGC
GACCGUGCGGGCCGAAUGCAGCCAGGAUUGCGCGACGUGCAGCUACC
GCCUAGUGCGCCCGGCCGACAUCAACUUCCUGGCUUGCGUAAUGGAAU
GUGAAGGUAAACUGCCUUCUCUGAAAAUUUGGGAAACCUGCAAGGAGC
UCCUGCAGCUGUCCAAACCAGAGCUUCCUCAAGAUGGCACCAGCACCC
UCAGAGAAAAUAGCAAACCGGAAGAAAGCCAUUUGCUAGCCAAAAGGU
AUGGGGGCUUCAUGAAAAGGUAUGGAGGCUUCAUGAAGAAAAUGGAU
GAGCUUUAUCCCAUGGAGCCAGAAGAAGAGGCCAAUGGAAGUGAGAU
CCUCGCCAAGCGGUAUGGGGGCUUCAUGAAGAAGGAUGCAGAGGAGG
ACGACUCGCUGGCCAAUUCCUCAGACCUGCUAAAAGAGCUUCUGGAAA
CAGGGGACAACCGAGAGCGUAGCCACCACCAGGAUGGCAGUGAUAAU
GAGGAAGAAGUGAGCAAGAGAUAUGGGGGCUUCAUGAGAGGCUUAAA
GAGAAGCCCCCAACUGGAAGAUGAAGCCAAAGAGCUGCAGAAGCGAU
AUGGGGGCUUCAUGAGAAGAGUAGGUCGCCCAGAGUGGUGGAUGGAC
UACCAGAAACGGUAUGGAGGUUUCCUGAAGCGCUUUGCCGAGGCUCU
GCCCUCCGACGAAGAAGGCGAAAGUUACUCCAAAGAAGUUCCUGAAAU
GGAAAAAAGAUACGGAGGAUUUAUGAGAUUUUAAUAUCUUUUCCCACU
AGUGGCCCCAGGCCCCAGCAAGCCUCCCUCCAUCCUCCAGUGGGAAACUG
UUGAUGGUGUUUUAUUGUCAUGUGUUGCUUGCCUUGUAUAGUUGACUUC
AUUGUCUGGAUAACUAUACAACCUGAAAACUGUCAUUUCAGGUUCUGUG
CUCUUUUUGGAGUCUUUAAGCUCAGUAUUAGUCUAUUGCAGCUAUCUCG
UUUUCAUGCUAAAAUAGUUUUUGUUAUCUUGUCUCUUAUUUUUGACAAA
CAUCAAUAAAUGCUUACUUGUAUAUAGAGAUAAUAAACCUAUUACCCCA
AGUGCA
___ : 5'UUR, ___ : Exon, ___ : 3'UUR

mRNA may be synthesized according to any of various methods known in the art. For example, the mRNA according to the present invention may be synthesized by *in vitro* transcription (IVT). IVT is typically performed using a linear or circular DNA template containing a promoter, a pool of ribonucleotide triphosphates, a buffer system that may contain DTT and magnesium ions, and an appropriate RNA polymerase (e.g., T3, T7, or SP6 RNA polymerase), as well as DNase I, pyrophosphatase, and/or an RNase inhibitor. Conditions may vary depending on the application.

The mRNA according to the present invention may be purified so as to be free from reagents, that is, impurities.

In some embodiments, using the present invention, mRNA containing one or more modifications that typically enhance stability may be purified. In some embodiments, the one or more modifications are selected from modified nucleotides, modified sugar-phosphate backbones, and 5' and/or 3' untranslated regions. In some embodiments, unmodified *in vitro*-synthesized mRNA may be purified using the present invention.

The mRNA may be modified to enhance stability. Modification of the mRNA may include, for example, modification of nucleotides of the RNA. Accordingly, the modified mRNA according to the present invention may include, for example, backbone modifications, sugar modifications, or base modifications. In some embodiments, an antibody-encoding mRNA (e.g., heavy chain- and light chain-encoding mRNA) may include purines (adenine (A), guanine (G)) or pyrimidines (thymine (T), cytosine (C), uracil (U)), and modified nucleotide analogs or derivatives of purines and pyrimidines, such as, for example, 1-methyladenine, 2-methyladenine, 2-methylthio-N6-isopentenyladenine, N6-methyladenine, N6-isopentenyladenine, 2-thiocytosine, 3-methylcytosine, 4-acetylcytosine, 5-methylcytosine, 2,6-diaminopurine, 1-methylguanine, 2-methylguanine, 2,2-dimethylguanine, 7-methylguanine, inosine, 1-methylinosine, pseudouracil (5-uracil), dihydrouracil, 2-thiouracil, 4-thiouracil, 5-carboxymethylaminomethyl-2-thiouracil, 5-(carboxyhydroxymethyl)-uracil, 5-fluorouracil, 5-bromouracil, 5-carboxymethylaminomethyl-uracil, 5-methyl-2-thiouracil, 5-methyluracil, N-uracil-5-oxyacetic acid methyl ester, 5-methylaminomethyl-uracil, 5-methoxyaminomethyl-2-thiouracil, 5'-methoxycarbonylmethyl-uracil, 5-methoxy-uracil, uracil-5-oxyacetic acid methyl ester, uracil-5-oxyacetic acid (v), 1-methyl-pseudouracil, queuosine, β-D-mannosyl-queuosine, wybutoxosine, phosphoramidate, phosphorothioate, peptide nucleotides, methylphosphonate, 7-deazaguanosine, 5-methylcytosine, and inosine, but are not limited thereto, and may be synthesized from naturally occurring nucleotides and/or nucleotide analogs (modified nucleotides).

In one embodiment, the nucleic acid includes a 5' untranslated region (UTR) located upstream of a nucleic acid encoding the PENK protein, and a 3' untranslated region (UTR) located downstream of the coding region, wherein the 5' UTR may be included in a gene construct including a coronavirus leader sequence.

A SARS-CoV-2 leader sequence and an intergenic sequence, which may act as a 5' untranslated region (UTR), may be linked, followed immediately by the coding sequence of a gene to be expressed, that is, a nucleic acid encoding the PENK protein, and further linked to a SARS-CoV-2 3' UTR sequence and a poly(A) sequence, and the resulting construct may be introduced into cells.

In this case, for expression of mRNA encoding the desired PENK protein *in vitro*, a promoter sequence (e.g., T7, SP6, etc.) may be linked to the 5' end of the sequence and mRNA encoding the PENK protein may be produced by an *in vitro* transcription (IVT) method.

Specifically, the present invention provides a gene construct including: a coding region for expression of mRNA encoding the PENK protein; a 5' untranslated region (UTR) located upstream of the coding region; and a 3' untranslated region (UTR) located downstream of the coding region, wherein the 5' UTR includes a coronavirus leader sequence.

With respect to the leader sequence, among coronaviruses, genomic RNA and transcriptionally expressed subgenomic mRNAs commonly share a leader sequence of approximately 72 to 77 bp at the 5' end, which is a distinctive characteristic of coronaviruses and represents one of the most abundant viral targets present in infected cells. This is because all coronavirus subgenomic RNAs exhibit a leader-joining phenomenon in which a leader sequence of approximately 72 bp, derived from the 5' end of the genomic RNA, is joined to the 5' end of each subgenomic RNA. Accordingly, the leader sequence has the highest copy number among viral genes within cells, followed by the subgenomic RNA encoding the N protein, which has the next highest copy number.

The RNA includes, for example, mRNA. RNA is an abbreviation for ribonucleic acid. RNA is a nucleic acid molecule, a polymer composed of nucleotides. Nucleotides are usually adenosine-monophosphate, uridine-monophosphate, guanosine-monophosphate and cytidine-monophosphate monomers linked to each other through a so-called "backbone". The backbone is formed by a phosphodiester bond between the sugar, i.e., ribose, and the phosphate moiety of the adjacent monomer. A specific consecutive sequence of monomers is called an "RNA sequence". Usually, RNA may be obtained, for example, within cells, by transcription of a DNA sequence. In eukaryotic cells, transcription is typically performed within the nucleus or mitochondria. *In vivo*, mRNA, messenger RNA, is transcribed from DNA. For example, processing of RNA in eukaryotic cells involves a variety of other post-transcriptional modifications such as splicing, 5'-capping, polyadenylation, and exports from the nucleus or mitochondria and the like. Messenger RNA usually provides a nucleotide sequence that may be translated into the amino acid sequence of a specific peptide or protein. Typically, mRNA includes a 5'-cap, a 5'-UTR, an open reading frame, a 3'-UTR and a poly(A) sequence. Aside from messenger RNA, there are several non-coding forms of RNA that may be involved in the regulation of transcription and/or translation.

The 5'-UTR is located at the 5' end (i.e., "upstream") of an open reading frame. The 5'-UTR starts at the transcription start site and ends at the nucleotide before the start codon of the open reading frame. The 5'UTR may contain elements that regulate gene expression, for example ribosome-binding sites. The 5'UTR may be modified post-transcriptionally, for example by addition of a 5'-cap. 5'UTR corresponds to the sequence of the mature mRNA located between the 5'-cap and the start codon.

Specifically, the 5' untranslated region (UTR) may include a sequence of SEQ ID NO. 2.

[SEQ ID NO. 2]
ATTAAAGGTTTATACCTTCCCAGGTAACAAACCAACCAACTTTCGATCTCTTG
TAGATCTGTTCTCTAAACGAACAAACTAAA

The 3'UTR is typically a portion of mRNA located between the protein-coding region of the mRNA (i.e. the open reading frame) and the poly(A) sequence. The 3'UTR of mRNA is not translated into amino acid sequence. 3'UTR sequences are usually encoded by genes, which are each transcribed into mRNA during the gene expression process. The genomic sequence is first transcribed into mRNA containing an optional intron. The mRNA then undergoes steps such as 5' capping, splicing and modification of the 3'-terminal, such as polyadenylation of the 3'-terminal, and optional endo- or exonuclease digestion. The 3'UTR is located immediately next to the stop codon 3' of the protein coding region and includes the nucleotide immediately next to the 5' poly(A) sequence.

The 3' untranslated region (UTR) may include the 3' UTR sequence of SARS-CoV-2 and may include the sequence of SEQ ID NO: 3.

[SEQ ID NO. 3]
CAATCTTTAATCAGTGTGTAACATTAGGGAGGACTTGAAAGAGCCACCACAT
TTTCACCGAGGCCACGCGGAGTACGATCGAGTGTACAGTGAACAATGCTAGG
GAGAGCTGCCTATATGGAAGAGCCCTAATGTGTAAAATTAATTTTAGTAGTGC
TATCCCCATGTGATTTTAATAGCTTCTTAGGAGAATGAC

The 3' untranslated region (UTR) typically has a sequence of several nucleotide triplets that may be translated into a peptide or protein. The open reading frame preferably includes a start codon, i.e. a combination of three subsequent nucleotides, usually encoding the amino acid methionine (ATG or AUG) at the 5'-terminal thereof, and a subsequent region usually having a length that is a multiple of 3 nucleotides. The ORF is preferably terminated by a stop codon (e.g., TAA, TAG, TGA). This is the only stop codon in the open reading frame. Therefore, in the context of the present invention, the open reading frame preferably starts with a start codon (e.g., ATG or AUG) and preferably stops with a stop codon (e.g., TAA, TGA, or TAG or UAA, UAG, UGA, respectively), and is a nucleotide sequence including three nucleotides. The open reading frame may be isolated or incorporated into longer nucleic acid sequences, such as vectors or mRNAs. The open reading frame may also be called a "protein coding region". Specifically, according to the present invention, a nucleic acid encoding a PENK protein includes the sequence of SEQ ID NO: 1.

According to the present invention, the gene construct may further include a promoter and/or poly(A) sequence.

The promoter may be located upstream of the 5' untranslated region (UTR). The promoter may include elements necessary for transcription, such as an RNA polymerase promoter. The promoter may include a phage RNA polymerase promoter such as SP6 or T7, preferably a T7 promoter encoding an mRNA sequence.

The length of the poly(A) sequence may vary. For example, the poly(A) sequence may be about 20 adenine nucleotides to about 300 adenine nucleotides, preferably about 40 to about 200 adenine nucleotides, such as 60, 70, 80, 90 or 100 adenine nucleotides, more preferably about 50 to about 100 adenine nucleotides, or about 20 adenine nucleotides to about 400 adenine nucleotides.

Specifically, in an embodiment according to the present invention, the poly(A) sequence may have a length of 65 nucleotides.

The poly(A) sequence may be located downstream of the 3' untranslated region (UTR). For example, the poly(A) sequence may be linked directly or through a linker, for example, through a linker of 1 to 50 nucleotides, preferably 1 to 20 nucleotides, or through a stretch of 2, 4, 6, 8, 10, and 20 nucleotides.

In a specific embodiment, the present invention may include a genetic construct including the sequence represented by SEQ ID NO: 4.

[SEQ ID NO: 4]

TAATACGACTCACTATAGGGATTAAAGGTTTATACCTTCCCAGGTAACAAACC
AACCAACTTTCGATCTCTTGTAGATCTGTTCTCTAAACGAACAAACTAAAATG
GCGCGGTTCCTGACACTTTGCACTTGGCTGCTGTTGCTCGGCCCCGGGCTCC
TGGCGACCGTGCGGGCCGAATGCAGCCAGGATTGCGCGACGTGCAGCTACC
GCCTAGTGCGCCCGGCCGACATCAACTTCCTGGCTTGCGTAATGGAATGTGA
AGGTAAACTGCCTTCTCTGAAAATTTGGGAAACCTGCAAGGAGCTCCTGCAG
CTGTCCAAACCAGAGCTTCCTCAAGATGGCACCAGCACCCTCAGAGAAAATA
GCAAACCGGAAGAAAGCCATTTGCTAGCCAAAAGGTATGGGGGCTTCATGA
AAAGGTATGGAGGCTTCATGAAGAAAATGGATGAGCTTTATCCCATGGAGCC
AGAAGAAGAGGCCAATGGAAGTGAGATCCTCGCCAAGCGGTATGGGGGCTT
CATGAAGAAGGATGCAGAGGAGGACGACTCGCTGGCCAATTCCTCAGACCT
GCTAAAAGAGCTTCTGGAAACAGGGGACAACCGAGAGCGTAGCCACCACCA
GGATGGCAGTGATAATGAGGAAGAAGTGAGCAAGAGATATGGGGGCTTCATG
AGAGGCTTAAAGAGAAGCCCCCAACTGGAAGATGAAGCCAAAGAGCTGCA
GAAGCGATATGGGGGCTTCATGAGAAGAGTAGGTCGCCCAGAGTGGTGGAT
GGACTACCAGAAACGGTATGGAGGTTTCCTGAAGCGCTTTGCCGAGGCTCTG
CCCTCCGACGAAGAAGGCGAAAGTTACTCCAAAGAAGTTCCTGAAATGGAA
AAAAGATACGGAGGATTTATGAGATTTTAACAATCTTTAATCAGTGTGTAACA
TTAGGGAGGACTTGAAAGAGCCACCACATTTTCACCGAGGCCACGCGGAGT
ACGATCGAGTGTACAGTGAACAATGCTAGGGAGAGCTGCCTATATGGAAGAG
CCCTAATGTGTAAAATTAATTTTAGTAGTGCTATCCCCATGTGATTTTAATAGCT
TCTTAGGAGAATGACAAAAAAAAAAAAAAAAAAAAAAAAAAAAAAAAAAAAAA
AAAAAAAAAAAAAAAAAAAAAAAAAAAAAAAAATGCAT

The composition may further contain a delivery vehicle, specifically an acceptable carrier, for delivering the nucleic acid, e.g., mRNA expressed or synthesized from the nucleic acid.

Based on this, in another aspect, the present invention is directed to a composition for preventing or treating bladder cancer including a nucleic acid encoding a PENK (proenkephalin) protein and a carrier.

In another aspect, the present invention is directed to a method of preventing or treating bladder cancer including administering to a subject a nucleic acid encoding a PENK (proenkephalin) protein and a carrier.

In another aspect, the present invention is directed to a use of a nucleic acid encoding a PENK (proenkephalin) protein and a carrier for the preparation of a composition for preventing or treating bladder cancer.

The mRNA may be delivered via nanoparticles. For example, the mRNA may be delivered via gold nanoparticles.

The surface of the gold nanoparticle may be modified. The modification may be exemplified in detail in Acc Chem Res. 2019 June 18; 52(6): 1496-1506. and Pharmaceutics 2021, 13, 900., etc., which are incorporated herein by reference.

Gold nanoparticles may be linked to the mRNA to form a complex with a cationic endosomal disruptive polymer and the complex may be delivered to cells (Nature Biomedical Engineering volume 1, pages 889-901 (2017)). The cationic endosomal disruptive polymer is, for example, polyethylene imine, poly(arginine), poly(lysine), poly(histidine), poly-[2-{(2-aminoethyl)amino}-ethyl-aspartamide] (pAsp(DET)), a block co-polymer of poly(ethylene glycol) (PEG) and poly(arginine), a block co-polymer of PEG and poly(lysine), or a block co-polymer of PEG and poly{N-[N-(2-aminoethyl)-2-aminoethyl]aspartamide} (PEG-pAsp(DET)).

In some cases, gold particles surface-modified with arginine may be used.

Gold particles modified with arginine may be assembled with a nuclease or a polynucleotide encoding the same and/or a cleavage factor, or a polynucleotide encoding the same. As a result, the result fuses with the membrane of the target cell and then moves into the cytoplasm (ACS Nano. 2017, 11:2452-2458).

Expressed mRNA may be delivered through liposomes, lipid nano-particles (LNPs), or various nanoparticles. Liposomes or LNPs contain cationic lipids, non-cationic lipids, or neutral lipids, and may further contain other lipids such as PEG (polyethylene glycol) or cholesterol. These mRNA delivery systems are described in detail in U.S. Patent Publication Nos. 2018/0311176, 2019/0032051, and 2021/0046192, International Patent Publication Nos. WO 2018/081480, WO 2020/097540, WO 2020/097548, and WO 2021/007278, and the like, which are incorporated herein by reference.

The cationic lipid is for example lipofectamine.

The cationic lipid is exemplified in detail in US Patent Publication Nos. 2018/0311176 and 2019/0032051, and the like, and is, for example, N,N-dioleyl-N,N-dimethylammonium chloride (DODAC), N,N-distearyl-N,N-dimethylammonium bromide (DDAB), N-(I-(2,3-dioleyloxy)propyl)-N,N,N-trimethylammonium chloride (DOTAP), N-(I-(2,3-dioleyloxy)propyl)-N,N,N-trimethylammonium chloride (DOTMA), N,N-dimethyl-2,3-dioleyloxy)propylamine (DODMA), 1,2-dilinoleyloxy-N,N-dimethylaminopropane (DLinDMA), 1,2-dilinolenyloxy-N,N-dimethylaminopropane (DLenDMA), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA·Cl), 1,2-dilinoleoyl-3-trimethylaminopropane chloride salt (DLin-TAP·Cl), 1,2-dilinoleyloxy-3-(N-methylpiperazino)propane (DLin-MPZ), or 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 1,2-dilinolenyloxy-N,N-dimethylaminopropane (DLinDMA), 2,2-dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (Tech G1), 2,2-dilinoleyl-4-dimethylaminoethyl-[1,3]-dioxolane, β-L-arginyl-2,3-L-diaminopropionic acid-N-palmityl-N-oleylamide trihydrochloride, N',N'-dioctadecyl-N-4,8-diaza-10-aminodecanoylglycine amide [71], 1,2-dilinoleyloxy-3-dimethylaminopropane, DLin-KC2-DMA, amino lipid 2,2-dilinoleyl-4-dimethylaminoethyl-[1,3]-dioxolane (DLin-KC2-DMA, 1), 1,2-distearloxy-N,N-dimethylaminopropane (DSDMA), dilinoleylmethyl-4-dimethylaminobutyrate (DLin-MC3-DMA), DLin-D-DMA, C12-200, 98N12-5, (20Z,23Z)-N,N-dimethylnonacosa-20,23-dien-10-amine, (17Z,20Z)-N,N-dimemylhexacosa-17,20-dien-9-amine, (1Z,19Z)-N5N-dimethylpentacosa-16,19-dien-8-amine, (13Z,16Z)-N,N-dimethyldocosa-13,16-dien-5-amine, (12Z,15Z)-N,N-dimethylhenicosa-12,15-dien-4-amine, (14Z,17Z)-N,N-dimethyltricosa-14,17-dien-6-amine, (15Z,18Z)-N,N-dimethyltetracosa-15,18-dien-7-amine, (18Z,21Z)-N,N-dimethylheptacosa-18,21-dien-10-amine, (15Z,18Z)-N,N-dimethyltetracosa-15,18-dien-5-amine, (14Z,17Z)-N,N-dimethyltricosa-14,17-dien-4-amine, (19Z,22Z)-N,N-dimethyloctacosa-19,22-dien-9-amine, (18Z,21Z)-N,N-dimethylheptacosa-18,21-dien-8-amine, (17Z,20Z)-N,N-dimethylhexacosa-17,20-dien-7-amine, (16Z,19Z)-N,N-dimethylpentacosa-16,19-dien-6-amine, (22Z,25Z)-N,N-dimethylhentriaconta-22,25-dien-10-amine, (21Z,24Z)-N,N-dimethyltriaconta-21,24-dien-9-amine, (18Z)-N,N-dimethylheptacos-18-en-10-amine, (17Z)-N,N-dimethylhexacos-17-en-9-amine, (19Z,22Z)-N,N-dimethyloctacosa-19,22-dien-7-amine, N,N-dimethylheptacosan-10-amine, (20Z,23Z)-N-ethyl-N-methylnonacosa-20,23-dien-10-amine, 1-[(11Z,14Z)-1-nonylicosa-11,14-dien-1-yl]pyrrolidine, (20Z)-N,N-dimethylheptacos-20-en-10-amine, (15Z)-N,N-dimethyleptacos-15-en-10-amine, (14Z)-N,N-dimethylnonacos-14-en-10-amine, (17Z)-N,N-dimethylnonacos-17-en-10-amine, (24Z)-N,N-dimethyltritriacont-24-en-10-amine, (20Z)-N,N-dimethylnonacos-20-en-10-amine, (22Z)-N,N-dimethylhentriacont-22-en-10-amine, (16Z)-N,N-dimethylpentacos-16-en-8-amine, (12Z,15Z)-N,N-dimethyl-2-nonylhenicosa-12,15-dien-1-amine, (13Z,16Z)-N,N-dimethyl-3-nonyldocosa-13,16-dien-1-amine, N,N-dimethyl-1-[(1S,2R)-2-octylcyclopropyl]heptadecan-8-amine, 1-[(1S,2R)-2-hexylcyclopropyl]-N,N-dimethylnonadecan-10-amine, N,N-dimethyl-1-[(1S,2R)-2-octylcyclopropyl]nonadecan-10-amine, N,N-dimethyl-21-[(1S,2R)-2-octylcyclopropyl]henicosan-10-amine,N,N-dimethyl-1-[(1S,2S)-2-{[(1R,2R)-2-pentylcyclopropyl]methyl}cyclopropyl]nonadecan-10-amine, N,N-dimethyl-1-[(1S,2R)-2-octylcyclopropyl]hexadecan-8-amine, N,N-dimethyl-[(1R,2S)-2-undecylcyclopropyl]tetradecan-5-amine, N,N-dimethyl-3-{7-[(1S,2R)-2-octylcyclopropyl]heptyl}dodecan-1-amine, 1-[(1R,2S)-2-heptylcyclopropyl]-N,N-dimethyloctadecan-9-amine, 1-[(1S,2R)-2-decylcyclopropyl]-N,N-dimethylpentadecan-6-amine, N,N-dimethyl-1-[(1S,2R)-2-octylcyclopropyl]pentadecan-8-amine, R-N,N-dimethyl-1-[(9Z,12Z)-octadeca-9,12-dien-1-yloxy]-3-(octyloxy)propa-n-2-amine, S-N,N-dimethyl-1-[(9Z,12Z)-octadeca-9,12-dien-1-yloxy]-3-(octy-loxy)propan-2-amine, 1-{2-[(9Z,12Z)-octadeca-9,12-dien-1-yloxy]-1-Roctyloxy)methyl]ethyl}pyrro-lidine, (2S)-N,N-dimethyl-1-[(9Z,12Z)-octadeca-9,12-dien-1-yloxy]-3-[(5Z)-oct-5-en-1-yloxy]propan-2-amine, 1-{2-[(9Z,12Z)-octadeca-9,12-dien-1-yloxy]-1-[(octyloxy)methyl]ethyl}azet-idine, (2S)-1-(hexyloxy)-N,N-dimethyl-3-[(9Z,12Z)-octadeca-9,12-dien-ylo-xy]propan-2-amine, (2S)-1-(heptyloxy)-N,N-dimethyl-3-[(9Z,12Z)-octadeca-9,12-dien-1-yloxy]pr-opan-2-amine, N,N-dimethyl-1-(nonyloxy)-3-[(9Z,12Z)-octadeca-9,12-dien-1-yloxy]propan-2-amine, N,N-dimethyl-1-[(9Z)-octadec-9-en-1-yloxy]-3-(octyloxy)propan-2-amine, (2S)-N,N-dimethyl-1-[(6Z,9Z,12Z)-octadeca-6,9,12-trien-1-yloxy]-3-(o-ctyloxy)propan-2-amine, (2S)-1-[(11Z,14Z)-icosa-11,14-dien-1-yloxy]-N,N-dimethyl-3-(pentyloxy)propa-n-2-amine, (2S)-1-(hexyloxy)-3-[(11Z,14Z)-icosa-11,14-dien-1-yloxy]-N,N-di-methylpropan-2-amine, 1-[(11Z,14Z)-icosa-11,14-dien-1-yloxy]-N,N-dimethyl-3-(octyloxy)propan-2-amine, 1-[(13Z,16Z)-docosa-13,16-dien-1-yloxy]-N,N-dimethyl-3-(octyloxy)propan-2-amine, (2S)-1-[(13Z,16Z)-docosa-13,16-dien-1-yloxy]-3-(hexyloxy)-N,N-dimethylpro-pan-2-amine, (2S)-1-[(13Z)-docos-13-en-1-yloxy]-3-(hexyloxy)-N,N-dimethylpropan-2-amine, 1-[(13Z)-docos-13-en-1-yloxy]-N,N-dimethyl-3-(octyloxy)propan-2-amine, 1-[(9Z)-hexadec-9-en-1-yloxy]-N,N-dimethyl-3-(octyloxy)propan-2-amine, (2R)-N,N-dimethyl-H(1-metoyloctyl)oxy]-3-[(9Z,12Z)-octadeca-9,12-dien-1-yloxy]propan-2-amine, (2R)-1-[(3,7-dimethyloctyl)oxy]-N,N-dimethyl-3-[(9Z,12Z)-octadeca-9,12-di-en-1-yloxy]propan-2-amine, N,N-dimethyl-1-(octyloxy)-3-({8-[(1S,2S)-2-{[(1R,2R)-2-pentylcyclopropyl]-methyl}cyclopropyl]octyl}oxy)propan-2-amine, N,N-dimethyl-1-{[8-(2-oclylcyclopropyl)octyl]oxy}-3-(octyloxy)propan-2-amine and (11E,20Z,23Z)-N,N-dimethylnonacosa-11,20,2-trien-10-amine, 5-carboxyspermylglycine dioctaoleoylamide (DOGS), dipalmitoylphosphatidylethanolamine 5-carboxyspermyl-amide (DPPES), 1,2-dimyristyloxypropyl-3-dimethyl-hydroxy ethyl ammonium bromide (DMRIE), DMRIE-HP, Lipofectamine (DOSPA), 3b-(N-(N',N'-dimethylaminoethane)-carbamoyl)cholesterol ("DC-Choi"), N-(1,2-dimyristyloxyprop-3-yl)-N,N-dimethyl-N-hydroxyethyl ammonium bromide ("DMRIE"), 1,2-dioleoyl-3-dimethylammonium-propane (DODAP), DMDMA, cationic lipid-based transfection reagents TransIT-TKO, LIPOFECTIN, Lipofectamine, OLIGOFECTAMINE or DHARMAFECT, DSDMA, DODMA, DLinDMA, DLenDMA, gamma-DLenDMA, DLin-K-DMA, DLin-K-C2-DMA (also known as DLin-C2K-DMA, XTC2, and C2K), DLin-K-C3-DM A, DLin-K-C4-DMA, DLen-C2K-DMA, y-DLen-C2K-DMA, DLin-M-C2-DMA (also known as MC2), DLin-M-C3-DMA (also known as MC3) or (DLin-MP-DMA)(also known as 1-B11), or a mixture thereof, but is not limited thereto.

The non-cationic lipid is exemplified in detail in US Patent Publication Nos. 2018/0311176 and 2019/0032051, and is, for example, phosphatidylglycerol, cardiolipin, diacylphosphatidylserine, diacylphosphatidic acid, N-dodecanoyl phosphatidylethanolamine, N-succinyl phosphatidylethanolamine, N-glutaryl phosphatidylethanolamine, or lysyl phosphatidylglycerol. In some cases, the non-cationic lipid may be, for example, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyl oleoyl phosphatidylethanolamine (POPE), dioleoyl-phosphatidylethanolamine 4-(-maleimidomethyl)-cyclohexane-1-carboxylate (DOPE-mal), dipalmitoyl phosphatidyl ethanolamine (DPPE), dimyristoyl phosphoethanolamine (DMPE), distearoyl-phosphatidyl-ethanolamine (DSPE), 16-O-monomethyl PE, 16-O-dimethyl PE, 18-1-trans PE, 1-stearoyl-2-oleoyl-phosphatidylethanolamine (SOPE), cholesterol, phosphatidylglycerols, cardiolipins, diacyl phosphatidylserines, diacylphosphatidic acids, N-dodecanoyl phosphatidylethanolamines, N-succinyl phosphatidylethanolamines, N-glutaryl phosphatidylethanolamines, lysyl phosphatidylglycerols, or palmitoyl oleoyl phosphatidylglycerol (POPG), but is not limited thereto.

The neutral lipid is specifically exemplified in U.S. Patent Publication Nos. 2018/0311176, 2019/0032051, etc., and, for example includes, but is not limited to, diacylphosphatidylcholine, diacylphosphatidylethanolamine, ceramide, sphingomyelin, dihydrosphingomyelin, cephalin, or cerebrosides.

A PEG lipid may be contained to prevent aggregation of particles produced during mRNA delivery. The PEG lipid is specifically exemplified in US Patent Publication Nos. 2018/0311176 and 2019/0032051, and is, for example, PEG-diacylglycerol (DAG), a PEG-dialkyloxypropyl (DAA), a PEG-phospholipid, a PEG-ceramide (Cer), or a mixture thereof. As a non-limiting example, PLGA may be conjugated to a lipid-terminating PEG forming PLGA-DSPE-PEG, PEG lipid is selected from PEG-c-DOMG, 1,2-dimyristoyl-sn-glycerol, methoxypolyethylene glycol (PEG-DMG), 1,2-distearoyl-sn-glycerol, methoxypolyethylene glycol (PEG-DSG), PEG-c-DOMG, 1,2-distearoyl-sn-glycerol, methoxypolyethylene glycol (PEG-DSG), 1,2-dipalmitoyl-sn-glycerol, methoxypolyethylene glycol (PEG-DPG), PEG-lipid conjugates such as, e.g., PEG coupled to dialkyloxypropyls (e.g., PEG-DAA conjugates), PEG coupled to diacylglycerols (e.g., PEG-DAG conjugates), PEG coupled to cholesterol, PEG coupled to phosphatidylethanolamines, PEG conjugated to ceramides, cationic PEG lipids, polyoxazoline (POZ)-lipid conjugates, polyamide oligomers (e.g., ATTA-lipid conjugates), and a mixture thereof. The PEG may be PEG-dilauryloxypropyl (C12), PEG-dimyristyloxypropyl (C14), PEG-dipalmityloxypropyl (C16), PEG-distearyloxypropyl (C18), PEG-c-DOMG, PEG-DMG, or a mixture thereof, but is not limited thereto.

Peptides may be used for mRNA delivery. The peptide must have a cation that electrostatically interacts with an anionic phosphate group of the nucleic acid and may contain a positively charged amino acid that electrostatically interacts with the phosphate group. Details of peptides that may be used for mRNA delivery are described in AIMS Biophysics, 7(4): 323-338, which is incorporated herein by reference.

The peptide that may be used for mRNA delivery may include protamine. Protamine is a small nuclear protein rich in cationic arginine that contributes to the stability of DNA during testicular spermatogenesis, which can stabilize mRNA molecules and enable efficient delivery. The protamine-mRNA complex is described in detail in US Patent No. 9352028, which is incorporated herein by reference.

Cell-penetrating peptides (CPPs) may also be promising cationic molecules for delivery of mRNA. Amphipathic CPPs such as arginine-rich RALA peptide (WEARLARALARALARHLARALARALRACEA), RALA, LAH4 (KKALLALALHHLAHLALHLALALKKA), and LAH4-L1 (KKALLAHALHLLALLALHLAHALKKA) may be used to deliver mRNA molecules.

In some cases, peptides may be further used for mRNA delivery in addition to the liposome or LNP (lipid nanoparticle). The peptide functions to package nucleic acids and prevent DNA or RNA from being degraded intracellularly or extracellularly. Examples of such peptides are described in detail in US Patent Publication No. 2021/0170046, which is incorporated herein by reference, but are not limited thereto.

The composition may further contain at least one pharmaceutically acceptable carrier. The pharmaceutically acceptable carrier must be compatible with the active ingredient of the present invention, may be saline solution, sterile water, Ringer's solution, buffered saline solution, dextrose solution, maltodextrin solution, glycerol, ethanol, or a mixture thereof, and may further contain other general additives such as antioxidants, buffers, and bacteriostatic agents as needed. In addition, the composition may be prepared into an injectable formulation such as an aqueous solution, suspension, or emulsion by adding a diluent, dispersant, surfactant, binder and lubricant thereto. In particular, the composition is preferably formulated into a lyophilizate. The lyophilizate may be prepared using a method commonly known in the art to which the present invention pertains and may be optionally prepared using a stabilizer for lyophilization. Furthermore, the composition is preferably formulated depending on each disease or ingredient using an appropriate method in the art or a method disclosed in Remington's Pharmaceutical Science (Mack Publishing company, Easton PA).

The content and administration method of the active ingredients contained in the composition of the present invention may be determined by those skilled in the art based on the severity of the symptoms and disease of the patient. In addition, the composition may be formulated in various forms such as powders, tablets, capsules, solutions, injections, ointments, and syrups, and may be provided in unit-dose or multi-dose containers, such as sealed ampoules and bottles.

The composition of the present invention may be administered orally or parenterally. The route of administration of the composition according to the present invention includes, but is not limited to, for example, bronchial, oral, intravenous, intramuscular, intraarterial, intramedullary, intrathecal, intracardiac, transdermal, subcutaneous, intraperitoneal, intestinal, sublingual or topical administration. The dosage of the composition according to the present invention varies depending on the weight, age, gender, health conditions, and diet of the patient, administration time, method, excretion rate, or severity of the disease, and is easily determined by those skilled in the art. In addition, the composition of the present invention may be formulated into a suitable dosage form for clinical administration using known techniques.

The subject may be a subject suffering from bladder cancer. In addition, the subject may be a mammal, and preferably a human.

As used herein, the term "treatment" refers to any indication of success in any subjective or objective parameter, for example, relief; remission; reduction in production, pathology or condition of symptoms or damage that the patient is tolerable; slowing of degeneration or deterioration; production of the final regression point at which the patient is less debilitating; and the treatment or amelioration of an injury, pathology or condition including improvement of the patient's physical or mental well-being. Treatment or alleviation of symptoms may be based on objective or subjective parameters including physical examination, neuropsychiatric examination and/or psychological medical evaluation.

The term "effective amount" is generally an amount that is sufficient to reduce the severity or frequency of symptoms, eliminate the symptoms and underlying causes, prevent the occurrence or underlying causes of symptoms, or ameliorate or correct damage resulting from or related to a disease condition. In some embodiments, an effective amount is a therapeutically effective amount or a prophylactically effective amount. A "therapeutically effective amount" is an amount that is sufficient to correct disease conditions or symptoms, in particular, conditions or symptoms associated with disease conditions, or otherwise prevent, hinder, delay or reverse the progression of disease conditions or any other undesirable symptoms associated in any way with diseases. The prophylactically effective amount refers to an amount of a pharmaceutical composition that has an intended prophylactic effect, e.g., an effect of preventing or delaying the onset of a disease condition, or reducing the likelihood of onset (or recurrence) of disease conditions or related symptoms when administered to subjects.

Hereinafter, the present invention will be described in more detail with reference to examples. However, it will be obvious to those skilled in the art that these examples are provided only for illustration of the present invention and should not be construed as limiting the scope of the present invention.

### Example 1. PENK expression in bladder cancer cell lines according to methylation

In order to determine the correlation between expression of a target *PENK* protein and DNA methylation, the methylation level of a bladder cancer cell line, T24, was first analyzed by pyrosequencing. As a result, a higher level of methylation was observed in T24 cells compared to normal cells (FIG. 1(A)). Subsequently, mRNA and protein expression were analyzed by RT-PCR and Western blot, respectively. As a result, PENK expression was not detected (FIGS. 1(B) and 1(C)). Accordingly, it was primarily confirmed that high methylation suppresses mRNA and protein expression.

### Example 2. Preparation of PENK gene mRNA construct for expression of PENK protein suppressed by methylation

### 2-1. Preparation of IVT template for PENK mRNA expression

The *PENK* mRNA construct for *PENK* protein expression was prepared based on the mRNA construct construction method previously developed by the present inventors (International Patent Application Publication No. WO2023/063769). The IVT template sequence previously constructed using this method was identified and shown in the table below.

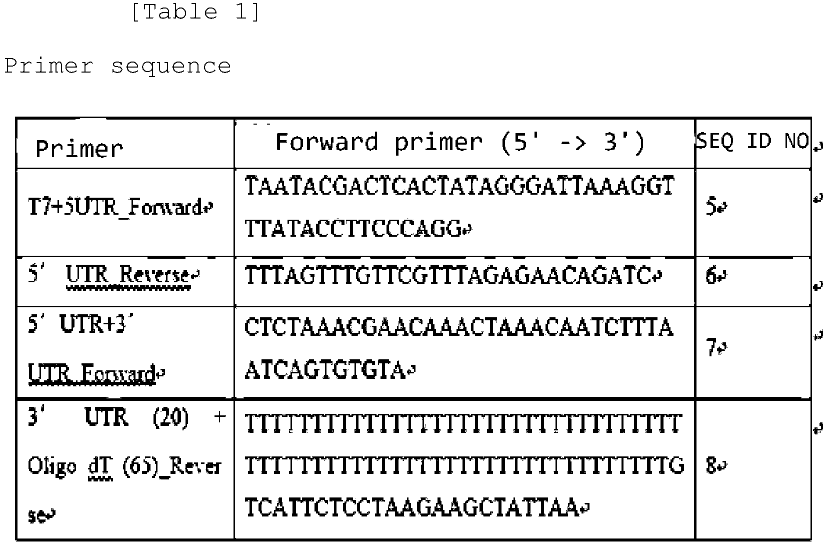

Furthermore, to construct the IVT template for *PENK* mRNA delivery, the PENK gene coding sequence was linked immediately after the 5' UTR of the SARS-CoV-2 N gene, followed by the SARS-CoV-2 3' UTR sequence and poly A sequence (65 nucleotides) via PCR (FIG. 2).

To synthesize the 5' UTR using 1 ng of the plasmid DNA for control RNA expression constructed above as a template, 10 pmoles of a forward primer (SEQ ID NO: 5), 10 pmoles of a reverse primer (SEQ ID NO: 6), and 10 µl of 2X pfu Master Mix (Biofact, Daejeon, South Korea) were added. To synthesize the 3' UTR, 10 pmoles of a forward primer (SEQ ID NO: 9), 10 pmoles of a reverse primer (SEQ ID NO: 8), and 10 µl of 2X pfu Master Mix (Biofact, Daejeon, South Korea) were added. The PCR (Applied Biosystem) reaction conditions were as follows: 95°C for 2 min (1 cycle); 95°C for 20 sec, 60°C for 40 sec, and 72°C for 1 min (30 cycles); 72°C for 5 min. The constructed PCR products were confirmed by electrophoresis on agarose gel (2%). The *PENK* gene was amplified using 1 ng of pCMV6-*PENK* containing the *PENK* gene as a template. 10 pmoles of a forward primer (SEQ ID NO: 10) in which the 3'-terminal sequence of the 5' UTR and the 5'-terminal sequence of the PENK gene were linked, 10 pmoles of a reverse primer (SEQ ID NO: 11) in which the 3'-terminal sequence of the PENK gene and the 5'-terminal sequence of the 3' UTR were linked, and 10 µl of 2X pfu Master Mix (Biofact, Daejeon, South Korea) was added. The PCR (Applied Biosystems) reaction conditions were as follows: 95°C for 2 min (1 cycle); 95°C for 20 sec, 60°C for 40 sec, and 72°C for 2 min (30 cycles); 72°C (5 min). The constructed PCR products were confirmed by electrophoresis on agarose gel (2%). The PCR method was used to link the T7 promoter sequence, 5' UTR sequence, *PENK* gene sequence, 3' UTR sequence, and 65 nucleotide poly A sequence. 100 pg of each PCR product constructed above was mixed in equal amounts and used as a template. 10 pmoles of the forward T7 promoter sequence primer (SEQ ID NO: 5), 10 pmoles of the reverse primer (SEQ ID NO: 8) including the 3'-terminal 20 nucleotides of the 3' UTR sequence linked to poly(A) of 65 nucleotides, and 10 µl of 2X pfu Master Mix (Biofact, Daejeon, South Korea) were added. The PCR (Applied Biosystems) reaction conditions were as follows: 95°C for 2 min (1 cycle); 95°C for 20 sec, 60°C for 40 sec, and 72°C for 2 min (30 cycles); 72°C (5 min). The PCR products were confirmed by agarose gel electrophoresis (2%). The PCR products were ligated to the pTOP Blunt V2 vector (Enzynomics, Daejeon, South Korea) and transformed into DH5α (Enzynomics, Daejeon, South Korea). After transformation, the products were plated on solid media (Bioloard, Daejeon, South Korea) containing ampicillin (50 µg/mL, Sigma Aldrich, USA) and incubated at 37°C for 16 hours. Plasmid DNA was extracted using the HiGene^{™} Plasmid Mini Prep Kit (Ver. 2.0) (Biofact, Daejeon, South Korea). The constructed IVT template sequence was confirmed by Sanger sequencing (SEQ ID NO: 4).

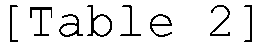

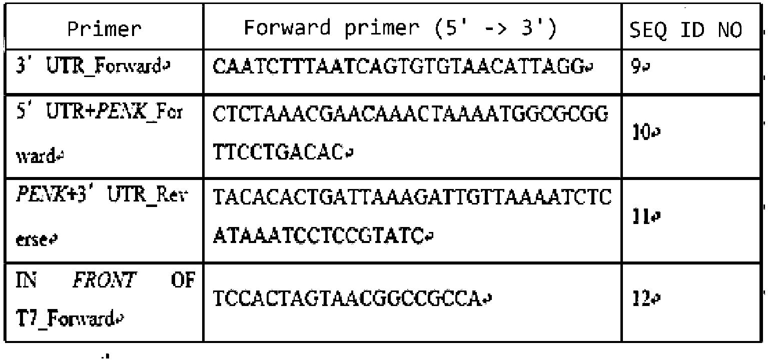

[SEQ ID NO: 4]

T7_5′ UTR_PENK_3′ UTR_poly A (65 mer)

TAATACGACTCACTATAGGGATTAAAGGTTTATACCTTCCCAGGTAAC
AAACCAACCAACTTTCGATCTCTTGTAGATCTGTTCTCTAAACGAACAAACTA
AAATGGCGCGGTTCCTGACACTTTGCACTTGGCTGCTGTTGCTCGGCCC
CGGGCTCCTGGCGACCGTGCGGGCCGAATGCAGCCAGGATTGCGCGAC
GTGCAGCTACCGCCTAGTGCGCCCGGCCGACATCAACTTCCTGGCTTGC
GTAATGGAATGTGAAGGTAAACTGCCTTCTCTGAAAATTTGGGAAACCT
GCAAGGAGCTCCTGCAGCTGTCCAAACCAGAGCTTCCTCAAGATGGCA
CCAGCACCCTCAGAGAAAATAGCAAACCGGAAGAAAGCCATTTGCTAGC
CAAAAGGTATGGGGGCTTCATGAAAAGGTATGGAGGCTTCATGAAGAAA
ATGGATGAGCTTTATCCCATGGAGCCAGAAGAAGAGGCCAATGGAAGTG
AGATCCTCGCCAAGCGGTATGGGGGCTTCATGAAGAAGGATGCAGAGG
AGGACGACTCGCTGGCCAATTCCTCAGACCTGCTAAAAGAGCTTCTGGA
AACAGGGGACAACCGAGAGCGTAGCCACCACCAGGATGGCAGTGATAA
TGAGGAAGAAGTGAGCAAGAGATATGGGGGCTTCATGAGAGGCTTAAA
GAGAAGCCCCCAACTGGAAGATGAAGCCAAAGAGCTGCAGAAGCGATA
TGGGGGCTTCATGAGAAGAGTAGGTCGCCCAGAGTGGTGGATGGACTA
CCAGAAACGGTATGGAGGTTTCCTGAAGCGCTTTGCCGAGGCTCTGCC
CTCCGACGAAGAAGGCGAAAGTTACTCCAAAGAAGTTCCTGAAATGGA
AAAAAGATACGGAGGATTTATGAGATTTTAACAATCTTTAATCAGTGTGTA
ACATTAGGGAGGACTTGAAAGAGCCACCACATTTTCACCGAGGCCACGCGG
AGTACGATCGAGTGTACAGTGAACAATGCTAGGGAGAGCTGCCTATATGGAA
GAGCCCTAATGTGTAAAATTAATTTTAGTAGTGCTATCCCCATGTGATTTTAAT
AGCTTCTTAGGAGAATGACAAAAAAAAAAAAAAAAAAAAAAAAAAAAAAA
AAAAAAAAAAAAAAAAAAAAAAAAAAAAAAAAAATGCAT

__: T7 leader, ....: 5′UTR, __: PENK, _ _: 3′UTR_poly A (65 mer)

### 2-2. Control mRNA and PENK mRNA structure verification

Based on Example 2-1, PCR was performed using 10 pmoles of the forward primer (SEQ ID NO: 12) corresponding to the forward sequence of the T7 promoter, 10 pmoles of the reverse primer (SEQ ID NO: 8) including the 3' UTR linked to polyA 65 of nucleotides, and 10 µl of 2X pfu Master Mix (Biofact, Daejeon, South Korea) in a total volume of 20 µL to produce each mRNA. The PCR reaction conditions were as follows: 95°C for 2 minutes (1 cycle); 95°C for 20 seconds, 60°C for 40 seconds, and 72°C for 2 minutes and 30 seconds (30 cycles); 72°C for 5 minutes. The amplified PCR products were purified using a Qiaquick^{®} Gel Extraction Kit (QIAGEN, Hilden, Germany) after electrophoresis on a 2% agarose gel and used as templates for intravitreal tyrosine spectroscopy (IVT). IVT was performed using the Hiscribe T7 ARCA mRNA kit (NEB) according to the manufacturer's instructions. 1 µg of the PCR product described above was used as a template, and 10 µl of 2X ARCA/NTP MIX and 2 µl of T7 polymerase were added and incubated at 37°C for 16 hours to allow mRNA synthesis and the addition of anti-reverse cap analog (ARCA) to the 5' end. After the reaction, 2 µl of DNase I was added and incubated at 37°C for 15 min to remove the DNA template. After the IVT reaction, the reaction solution was purified using the Monarch RNA Cleanup kit (NEB, Massachusetts, USA) and eluted in 50 µl of RNase-free distilled water. The purified RNA was quantified using NanoDrop and the mRNA product was confirmed by electrophoresis on an agarose gel (1%, 0.5X TBE) (FIG. 3). After electrophoresis, the size corresponded to the expected size.

### Example 3. Confirmation of PENK protein expression in human cell lines (Control vs. PENK)

### 3-1. Transfection and cell harvesting

The bladder cancer cell line T24 was seeded in 60 mm culture plates (SPL, Pocheon, Korea) at a density of 0.8 × 10⁶ cells per well and incubated for 24 hours in an incubator at 37°C under 5% CO₂. mRNA synthesized by an *in vitro* transcription (IVT) method was mixed with Lipofectamine MessengerMAX at a ratio of 1:1.5 (w:v) and transfected into the T24 cell line. After incubation for 24 hours at 37°C under 5% CO₂, cells were harvested. The harvested cells were subjected to protein extraction using Pro-Prep (iNtRON Biotechnology, Seongnam, South Korea) according to the manufacturer's instructions.

### 3-2. Confirmation of PENK protein expression and apoptotic function of PENK by Western Blot

Extracted proteins were separated by SDS-PAGE using Mini-PROTEAN tetra vertical electrophoresis cells (Bio-Rad, California, USA) and transferred onto a nitrocellulose membrane (Bio-Rad, California, USA). The membrane was blocked with 5% skim milk (Bio-Rad, Seoul, South Korea) for 1 hour, followed by incubation with a *PENK* polyclonal antibody (Invitrogen, Massachusetts, USA) diluted in 5% skim milk at 4°C for 16 hours. The membrane was washed three times with 1×TBST for 10 minutes each and was incubated with a mouse anti-rabbit IgG HRP secondary antibody (Santa Cruz Biotechnology, Texas, USA) diluted in 5% skim milk at room temperature for 2 hours, followed by three additional washes with 1 x TBST. The membrane was treated with a 1:1 mixture of luminol/enhancer solution and peroxide solution (Cyanagen, Bologna, Italy) for 1 minute at room temperature, and protein expression was visualized using a fusion solo X imaging system (Vilber, Eberhardzell, Germany) (FIG. 4(A)).

To determine the apoptotic function of *PENK*, protein expression levels of apoptosis-related factors PARP and Caspase-3 were also analyzed. Extracted proteins were subjected to SDS-PAGE using Mini-PROTEAN Tetra Vertical Electrophoresis Cell (Bio-rad, California, USA) and transferred to a nitrocellulose membrane, which was blocked with 5% skim milk for 1 hour. The membrane was incubated with anti-PARP (Cell Signaling Technology, Massachusetts, USA) and anti-Caspase-3 antibodies (Cell Signaling Technology, Massachusetts, USA), each diluted in 5% skim milk, at 4°C for 16 hours. The membrane was washed three times with 1 × TBST and was incubated with a mouse anti-rabbit IgG HRP secondary antibody at room temperature for 2 hours, followed by additional washing. Detection was performed as described above using the Fusion Solo X imaging system (FIG. 4(B)). This indicates that the *PENK* gene induces apoptosis more effectively than the control gene, thereby efficiently inhibiting proliferation of bladder cancer cells, suggesting its potential use as a therapeutic agent for bladder cancer.

### Example 4. Evaluation of inhibitory effects of PENK on cell proliferation and colony formation

### 4-1. Cell growth inhibition test

The bladder cancer cell line T24 was seeded in 24-well plates (SPL, Pocheon, Korea) at a density of 0.05 × 10⁶ cells per well and incubated for 24 hours at 37°C under 5% CO₂. mRNA synthesized by the IVT method was mixed with Lipofectamine MessengerMAX at a ratio of 1:1.5 (w:v) and transfected into T24 cells, followed by incubation at 37°C under 5% CO₂ for 24 hours. The cells were treated according to the manufacturer's instructions using a Cell Counting Kit-8 (Dojindo, Kumamoto, Japan) and incubated for 1 hour in a CO₂ incubator, and then absorbance of viable cells at 450 nm was measured using a Mobi microplate reader (Microdigital, Seoul, Korea). As a result, cell proliferation in cells transfected with PENK mRNA was reduced by approximately 37% compared to cells transfected with control mRNA (FIG. 5). These results demonstrate that the PENK gene effectively inhibits proliferation of bladder cancer cells and thus has potential applicability as a therapeutic agent for bladder cancer.

### 4-2. Evaluation of inhibitory effect on colony formation (colony formation assay)

The bladder cancer cell line T24 was seeded in 60 mm culture plates (SPL, Pocheon, Korea) at a density of 0.8 × 10⁶ cells per well and incubated for 24 hours at 37°C under 5% CO₂. mRNA synthesized by an *in vitro* transcription (IVT) method was mixed with Lipofectamine MessengerMAX at a ratio of 1:1.5 (w:v) and transfected into T24 cells. After incubation for 24 hours at 37°C under 5% CO₂, cells were harvested. The harvested cells were seeded into 6-well plates at a density of 3 x 10³ cells per well and incubated at 37°C under 5% CO₂ for 7 days. After colony formation, the culture medium in each well was removed and the cells were fixed with 100% methanol at room temperature for approximately 20 minutes. After cell fixation, the cells were washed with distilled water, stained with 0.5% crystal violet, and washed again with distilled water, and colony formation was evaluated. As a result, colony formation of cells transfected with the PENK gene was reduced by approximately 37% compared to cells transfected with a control gene (FIG. 6). These results indicate that the PENK gene effectively inhibits colony formation of bladder cancer cells and thus has potential use as a therapeutic agent for bladder cancer.

### Example 5. Evaluation of inhibitory effects of PENK on cell migration and invasion

### 5-1. Wound healing assay

The bladder cancer cell line T24 was seeded in 24-well plates (SPL, Pocheon, Korea) at a density of 0.05 × 10⁶ cells per well and incubated at 37°C under 5% CO₂ for 24 hours. mRNA synthesized by the IVT method was mixed with Lipofectamine MessengerMAX at a ratio of 1:1.5 (w:v) and transfected into T24 cells, followed by incubation at 37°C under 5% CO₂ for 24 hours. A uniform wound was then generated using a Scar^{™} Scratcher (SPL, Pocheon, Korea) and the cells were washed with PBS. The cells were further incubated for 24 hours and images before and after incubation were analyzed using ImageJ software to measure cell migration distances within the wound area. As a result, the wound healing ability of cells transfected with the *PENK* gene was reduced by approximately 40% at 24 hours compared to cells transfected with a control gene (FIG. 7). These results indicate that the *PENK* gene significantly reduces cell migration and wound healing ability, thereby effectively inhibiting proliferation of bladder cancer cells, and having potential applicability as a therapeutic agent for bladder cancer.

### 5-2. Transwell invasion assay

The bladder cancer cell line T24 was seeded in 24-well plates (SPL, Pocheon, Korea) at a density of 0.05 × 10⁶ cells per well and cultured for 24 hours at 37°C under 5% CO₂. mRNA synthesized by an *in vitro* transcription (IVT) method was mixed with Lipofectamine MessengerMAX at a ratio of 1:1.5 (w:v) and transfected into T24 cells. After incubation for 24 hours at 37°C under 5% CO₂, cells were harvested. First, the harvested cells were seeded into the upper chamber of a Matrigel-coated polycarbonate filter (Corning, New York, USA) at a density of 1 × 10⁴ cells per well in serum-free RPMI medium supplemented with 0.5% BSA, while RPMI medium containing serum was added to the lower chamber. The cells were then incubated at 37°C under 5% CO₂ for 24 hours. After incubation, the cells were fixed and stained using a Diff-Quik kit (Sysmex, Kobe, Japan), and invaded cells were counted under a microscope. As a result, Transwell invasion of cells transfected with the PENK gene was reduced by approximately 50% compared to cells transfected with a control gene (FIG. 8). These results indicate that the *PENK* gene regulates motility of bladder cancer cells, thereby significantly reducing cell migration and invasion, and having potential applicability as a therapeutic agent for bladder cancer.

### [Industrial applicability]

The present invention provides a composition for preventing or treating bladder cancer that exhibits inhibition of bladder cancer cell growth, induction of apoptosis, and suppression of cancer cell migration and invasion.

Although specific configurations of the present invention have been described in detail, those skilled in the art will appreciate that this detailed description is provided as preferred embodiments for illustrative purposes and should not be construed as limiting the scope of the present invention. Therefore, the substantial scope of the present invention is defined by the accompanying filed claims and equivalents thereto.

### [Sequence Listing Free Text]

An electronic file is attached.

## Claims

1. A composition for preventing or treating bladder cancer comprising a nucleic acid encoding a PENK (proenkephalin) protein.

2. The composition according to claim 1, wherein the nucleic acid comprises a sequence represented by SEQ ID NO: 1 or a nucleotide sequence having at least 90% identity thereto.

3. The composition according to claim 1, wherein the nucleic acid is DNA or RNA,
wherein, when the nucleic acid is RNA, thymidine(T) residues in the nucleotide sequence are replaced with urcil(U).

4. The composition according to claim 1, wherein the nucleic acid comprises:
a 5' untranslated region (UTR) located upstream of a coding regionencoding the PENK protein; and
a 3' untranslated region (UTR) located downstream of the coding region,
wherein the 5' untranslated region (UTR) is present in a gene construct comprising a coronavirus leader sequence.

5. The composition according to claim 4, wherein the 5' untranslated region (UTR) comprises a nucleotide sequence represented by SEQ ID NO: 2.

6. The composition according to claim 4, wherein the 3' untranslated region (UTR) comprises a nucleotide sequence represented by SEQ ID NO: 3.

7. The composition according to claim 4, wherein the gene construct further comprises a promoter and/or a poly(A) sequence.

8. The composition according to claim 1, wherein the composition comprises a gene construct comprising a nucleotide sequence represented by SEQ ID NO: 4.

9. A composition for preventing or treating bladder cancer, comprising: a nucleic acid encoding a PENK (proenkephalin) protein; and a pharmaceutically acceptable carrier.
